# EUROPEAN PATENT SPECIFICATION

(11) **EP 1 539 673 B1**
(45) Date of publication and mention of the grant of the patent: **03.10.2007**
(21) Application number: 03762669.4
(22) Date of filing: 09.07.2003
(51) Int. Cl.: C07C 213/08, C07D 307/46, C07D 333/22, C07C 225/12, C07C 221/00

(54) **PROCESS FOR THE PREPARATION OF N -MONOSUBSTITUTED BETA-AMINO ALCOHOLS**
VERFAHREN ZUR HERSTELLUNG VON N-MONOSUBSTITUIERTEN -AMINOALKOHOLEN
PROCEDE DE PRODUCTION DE BETA-AMINO ALCOOLS N-MONOSUBSTITUE

(30) Priority: 09.07.2002 EP 02015229
(43) Date of publication of application: 15.06.2005
(60) Divisional application: 07015051.1
(73) Proprietor: Lonza AG, 4052 Basel (CH)
(72) Inventor: MICHEL, Dominique, CH-3960 Sierre (CH)
(86) International application number: PCT/EP2003/007411
(87) International publication number: WO 2004/005239

(56) References cited:
- EP-A- 0 046 288
- EP-A- 0 457 559
- EP-A- 0 650 965
- F. F. BLICKE: "The Mannich reaction" ORGANIC REACTIONS, vol. I, 1942, pages 303-341, XP002218609
- KIYOSHI MATSUMOTO: "Synthese unter hohem Druck: Mannich-Reaktion....." ANGEWANDTE CHEMIE., vol. 94, no. 12, 1982, page 937 XP002218608 VCH VERLAGSGESELLSCHAFT, WEINHEIM., DE ISSN: 0044-8249
- CHEMICAL ABSTRACTS, vol. 56, no. 1, 1962 Columbus, Ohio, US; abstract no. 363g, G. I. DENIS ET AL.: "Alkylation of aromatic amines by the Mannich bases." page 363; column 1; XP002218610 & IZVEST. VYSSHIKH UCHEB. ZAVEDENII, KHIM. I KHIM. TEKHNOL., vol. 4, 1961, pages 426-428,
- CHEMICAL ABSTRACTS, vol. 52, no. 13, 1958 Columbus, Ohio, US; abstract no. 11067b, LEWIS W. NOBLES ET AL.: "Ketonic Mannich bases...." page 11067; column 1; XP002218611 & J. AM. PHARM. ASSOC., SCI. ED., vol. 67, 1958, pages 77-81,
- CHEMICAL ABSTRACTS, vol. 63, no. 8, 1965 Columbus, Ohio, US; abstract no. 9900g, R. LANDI-VITTORY ET AL.: "Derivatives of benzofuran and coumaran." page 9899; column 2; XP002218612 & FARMACO (PAVIA), vol. 18, no. 2, 1965, pages 109-118,
- CHEMICAL ABSTRACTS, vol. 67, no. 11, 1967 Columbus, Ohio, US; abstract no. 53946c, B. I. ARDASHEV ET AL.: "Synthesis of Beta-arylamino ketones of the furan series." page 5066; column 1; XP002218613 & KHIM. GETEROTSIKL. SOEDIN., vol. 1, 1967, pages 7-9,
- CHEMICAL ABSTRACTS, vol. 101, no. 13, 1984 Columbus, Ohio, US; abstract no. 110812c, SAAKYAN, A. M. ET AL.: "studies on the chlorination of organic compounds...." page 647; column 2; XP002218614 & ARM. KHIM. ZH., vol. 37, no. 4, 1984, pages 261-265,
- CHEMICAL ABSTRACTS, vol. 95, no. 1, 1981 Columbus, Ohio, US; abstract no. 6707u, AGARWAL, S. K. ET AL.: "Synthesis, characterization and screening of antibacterial activity of some new Mannich bases." page 635; column 1; XP002218615 & J. INDIAN CHEM. SOC., vol. 57, no. 12, 1980, pages 1240-1241,
- CHEMICAL ABSTRACTS, vol. 70, no. 9, 1969 Columbus, Ohio, US; abstract no. 37630b, TILAK, B. D. ET AL.: "Synthesis of nitrogen heterocyclics." page 331; column 1; XP002218616 & INDIAN J. CHEM., vol. 6, no. 8, 1968, pages 422-427,
- CHEMICAL ABSTRACTS, vol. 102, no. 1, 1985 Columbus, Ohio, US; abstract no. 6087e, XU, XIUJUAN ET AL.: "Mannich reaction with...." page 552; column 1; XP002218617 & HUAXUE XUEBAO, vol. 42, no. 7, 1984, pages 688-692,
- CHEMICAL ABSTRACTS, vol. 59, no. 2, 1963 Columbus, Ohio, US; abstract no. 1625b, N. SALDABOLS ET AL.: "New Mannich bases..." page 1625; column 1; XP002218618 & LATVIJAS PSR ZINATNU AKAD. VESTIS, KIM. SER., vol. 2, 1962, pages 309-310,
- W. LEWIS ET AL.: "Ketonic Mannich bases....." JOURNAL OF THE AMERICAN PHARMACEUTICAL ASSOCIATION, vol. 47, no. 2, 1958, pages 77-81, XP001115095

## Description

The invention relates to a process for the preparation of *N*-monosubstituted β-amino alcohols of formula 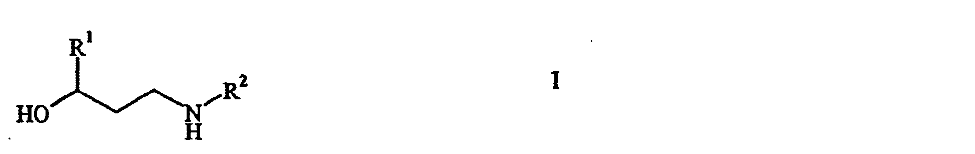 and/or an addition salt of a proton acid via direct synthesis of *N*-monosubstituted β-keto amines of formula 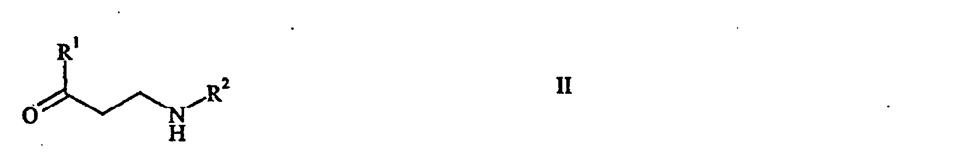 and/or an addition salt of a proton acid.

*N-*Monosubstituted β-amino alcohols of formula I like (*S*)-(-)-3-*N*-methylamino-1-(2-thienyl)-1-propanol (LY293628) are useful key intermediates and building blocks for the preparation of pharmaceutically active compounds like (*S*)-(+)-methyl-[3-(1-naphthyloxy)-3-(2-thienyl)-propyl]-amine ((*S*)-duloxetine) (Liu, H. et al., Chirality 12 (2000) 26-29), a potential neuro-active compound which strongly inhibits the serotonine and norephedrine uptake (Deeter, J. et al., Tetrahedron Lett. 31 (1990) 7101-7104).

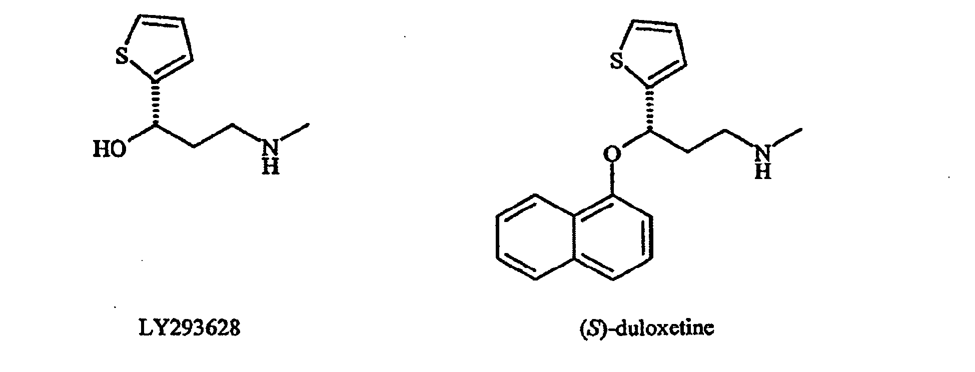

In the following the terms "amine" or "amines" include their corresponding addition salts of proton acids.

Direct preparation of *N*-monosubstituted β-keto amines of formula II establishes an alternative and economically advantageous source for industrial production of *N*-monosubstituted β-amino alcohols of formula I.

Compounds of formula II were first synthesized in 1922 by reacting ketones with formaldehyde and primary or secondary alkylamines in the presence of hydrochloric acid (Mannich, C. et al., Chem. Ber. 55 (1922) 356-365). In said reactions with primary alkylamines formation of hydrochlorides of tertiary β-keto amines of formula 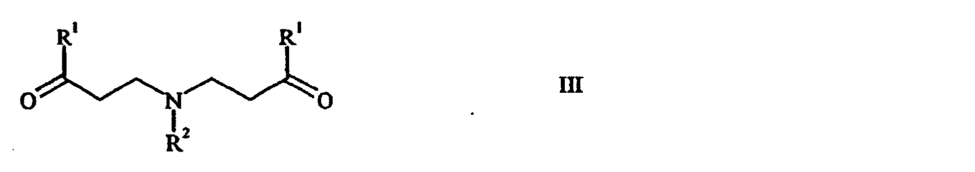 prevails over formation of hydrochlorides of secondary β-keto amines of formula II. These findings were supported by Blicke et al. (J. Am. Chem. Soc. 64 (1942) 451-454) and Becker et al. (Wiss. Z. Tech. Hochsch. Chem. Leuna-Merseburg. 11 (1969) 38-41).

According to Mannich et al. steam destillation of tertiary β-keto amines of formula III results in formation of secondary β-keto amines of formula II in fairly satisfactory yields, accompanied by vinyl compounds and other by-products.

In spite of the loss of more than 50 % of the starting compounds and due to lack of alternative processes this procedure is still used for the preparation of secondary β-keto amines.

Also from Blicke, F.F., Organic reactions, Vol. 1, Chapter 10, The Mannich Reaction, 1942, 303-341 is known that the Mannich reaction using primary amines leads to many side products.

Nobles, L.W. et al., J. Am. Pharm. Assoc., Sci. Ed., 67, 1958, 77-81 disclose among many compounds according to the compounds of formula II having a *N,N*-disubstituted amino group only one compound having a benzylamino group which is obtained in low yield.

Another drawback in presently known preparation methods of β-keto amines is the need of isolation of the desired intermediate compounds of formula II from unwanted by-products of formula III.

EP-A 457 559 and EP-A 650 965 disclose the preparation of *N,N*-dimethyl β-amino alcohols via Mannich-type reactions of methyl ketones with paraformaldehyde and dimethylamine followed by reduction of the carbonyl group. After reaction of the hydroxyl group affording alkyl or aryl ether derivatives one methyl radical is removed to obtain *N*-monosubstituted compounds which requires delicate and expensive reactions.

Only Becker et al. disclose some few examples with yields of about 60% of *N*-monomethyl β-keto amines using *N-*methylammonium oxalates as nitrogen source. Nevertheless, the process disclosed by Becker et al. is not advantageous because it strictly depends on the use of amino oxalates. In contrast to the free amines or corresponding hydrochlorides oxalates of primary amines are not commercially available and their preparation requires further synthesis and purification steps.
Using oxalates is also disadvantageous because it requires additional reduction equivalents in the next step, reducing the ketone intermediates to the title compounds.

None of the known processes for the production of *N*-monosubstituted β-amino alcohols of formula I and ether derivatives thereof includes, intends or concerns intermediate products comparable to *N*-monosubstituted β-keto amines of formula II of the present invention. Although still many efforts were made to find new preparation processes, the pathway of the present invention for direct synthesis of *N*-monosubstituted β-keto amines and subsequent reduction to *N*-monosubstituted β-amino alcohols is not yet disclosed.

The problem to be solved was to provide an alternative and efficient process for the synthesis of *N*-monosubstituted β-amino alcohols and derivatives thereof in high yields. Furthermore, the proposed process should provide high yields independently of steric aspects of the used amino or carbonyl compounds.

The problems mentioned above could be solved according to claim 1.

Starting with commercially available methyl ketones and primary amines and/or an addition salt of a proton acid, which were reacted with formaldehyde in the presence a solvent and optionally of a proton acid at a pressure above 1.5 bar *N*-monosubstituted β-Keto amines which could be directly reduced to the desired *N*-monosubstituted β-amino alcohols were obtained in high yields.

As a further advantage of the instant process high yields of *N*-monomethyl β-keto amines can be obtained by direct usage of methylamine hydrochloride which is easily available, cheap and, since it is a solid compound, easy to handle.

The present invention discloses a process for the preparation of a compound of formula 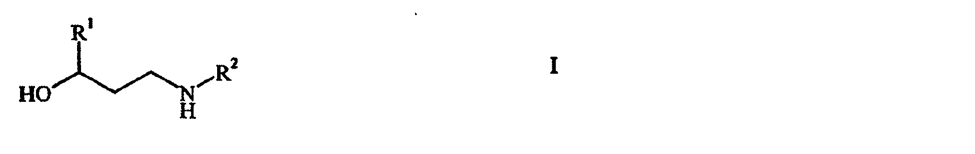 and/or an addition salt of a proton acid, wherein R¹ and R² independently represent alkyl, cycloalkyl, aryl or aralkyl, each being optionally further substituted with alkyl, alkoxy and/or halogen, which process comprises the steps of
a) reacting a mixture comprising
   (i) a methyl ketone of formula 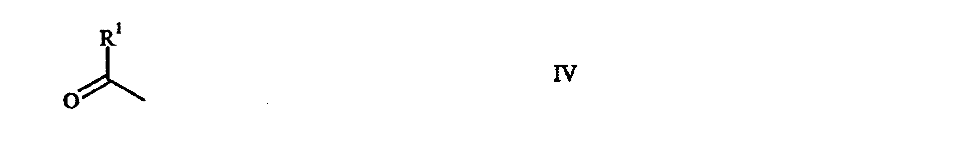 wherein R¹ is as defined above,
   (ii) a compound of formula

      H₂N-R² V

      and/or an addition salt of a proton acid, wherein R² is as defined above, and
   (iii) formaldehyde or a source of formaldehyde selected from the group consisting of formaldehyde in aqueous solution, 1,3,5-trioxane, paraformaldehyde and mixtures thereof, in the presence of
      a solvent selected from the group consisting of water, aliphatic alcohols, cycloaliphatic alcohols and mixtures thereof, and optionally a proton acid
   to afford a compound of formula 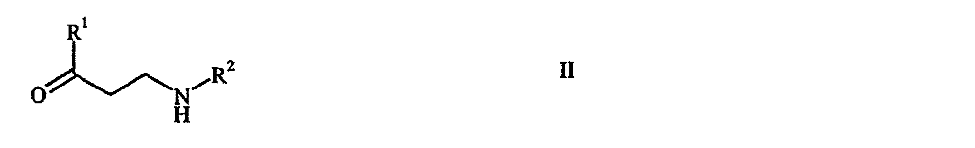 and/or an addition salt of a proton acid, and
b) reducing the carbonyl group of said β-keto amine to afford a compound of formula I, and/or an addition salt of a proton acid,
wherein the first step is carried out at a pressure above 1.5 bar.

In a preferred embodiment R¹ and R² can independently represent
linear or branched C₁₋₈ alkyl, C₃₋₈ cycloalkyl, phenyl, naphthyl, furanyl, benzofuranyl, thienyl, benzo[b]thienyl or aralkyl, wherein the alkyl moiety of the aralkyl residue is linear C₁₋₄ alkyl,
and the aryl moiety is selected from the group consisting of phenyl, naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F_{5.}

It is particularly preferred that R¹ represents furanyl or thienyl.
It is also particularly preferred that R² represents linear or branched C₁₋₈ alkyl. More particularly preferred R² represents methyl, ethyl, propyl, isopropyl, butyl, isobutyl or *tert-*butyl.

Preferably, the compound of formula V is used as a free amine and/or an addition salt of a proton acid. Particularly preferred are free amines, formates, acetates, oxalates, hydrochlorides, hydrobromides or mixtures thereof. More particularly preferred are free amines and/or hydrochlorides.

In a preferred embodiment the compound of formula V is present in an amount at least equimolar to that of the compound of formula IV. Particularly preferred the molar ratio of the compound of formula V to the compound of formula IV is between 1 and 2.

In a preferred embodiment the solvent comprises water, an aliphatic or cycloaliphatic alcohol or a mixture thereof.

Particularly preferred alcohols are linear or branched aliphatic C₁₋₁₂ alcohols, cycloaliphatic C₅₋₈ alcohols, di- and/or trimeric ethylene glycols or mono C₁₋₄ alkyl or acetyl derivatives thereof, each of said alcohols containing 1 to 3 hydroxy groups.

Examples for said alcohols are methanol, ethanol, propanol, isopropyl alcohol, butanol, isobutanol, *tert*-butanol, 2-butanol, 1-pentanol, 2-pentanol, 3-pentanol, 1-hexanol, 2-hexanol, cyclopentanol, cyclohexanol, 1,2-ethanediol, 1,2-propanediol, 1,2-butanediol, 2,3-butanediol, 1,4-butanediol, 1,2,3-propanetriol, 1,2,6-hexanetriol, diethylene glycol, diethylene glycol monomethyl ether, diethylene glycol monoethyl ether, diethylene glycol monobutyl ether, diethylene glycol monoacetate, triethylene glycol, triethylene glycol monomethyl ether, triethylene glycol monoethyl ether, triethylene glycol monobutyl ether and triethylene glycol monoacetate.

Preferably said alcohol is ethanol, propanol, isopropyl alcohol, butanol, isobutanol, *tert*-butanol, diethylene glycol or triethylene glycol.

The proton acid can be any organic or inorganic acid, the acid being preferably selected from the group consisting of formic acid, acetic acid, propionic acid, oxalic acid, malonic acid, benzoic acid, HF, HCl, HBr, HI, H₂SO₄ and H₃PO₄. In a preferred embodiment the proton acid can be an acidic salt of a polybasic organic or inorganic acid like monoalkali malonates, alkali hydrogensulfates, alkali hydrogenphosphates and alkali hydrogencarbonates.
More preferably the proton acid is selected from the group consisting of formic acid, acetic acid, propionic acid, oxalic acid, HCl and HBr, more preferably it is selected from the group consisting of formic acid, acetic acid, HCl and HBr.

Preferably reaction step a) is carried out either with added addition salts of amines or proton acids, since even distilled free β-amino ketones of formula II tend to decompose and form by-products while stored, whereas the corresponding additions salts can be stored over a longer period without decomposition. In the products, the ratio of free amine and its salt corresponds to the ratio of added addition salts of amines and proton acids to the whole amine amount during reaction step a).

In a preferred embodiment the pressure during reaction step a) is above 1.5 bar, More preferably the pressure is in the range of 1.5 to 10 bar and particularly preferred in the range of 1.5 to 5 bar.

In contrast to Becker et al. the inventive process generally allows direct preparation of *N*-monosubstitated β-keto amines and addition salts of proton acids thereof. The products obtained by the inventive process can be reduced or subsequently reacted without further conversion into other salts.

The present invention also provides a compound of formula 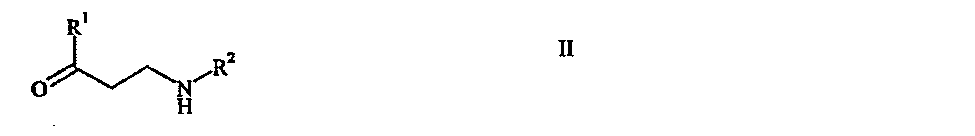 and its addition salts of proton acids,
wherein R¹ represents furanyl, benzofuranyl, isobenzofuranyl, thienyl or benzo[b]thienyl, each being optionally substituted with halogen, linear or branched C₁₋₄ alkyl, linear or branched C₁₋₄ alkoxy, C₃-₆ cycloalkyl, CF₃, C₂F₅, OCF₃ or OC₂F₅, and
wherein R² is selected from the group consisting of linear or branched C₁₋₈ alkyl, C₃₋₈ cycloalkyl, furanyl, benzofuranyl, thienyl, benzo[b]thienyl and aralkyl, wherein the alkyl moiety of the arakyl residue is linear C₁₋₄ alkyl, and the aryl moiety is selected from the group consisting of naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅,
with the exception of compounds wherein R¹ represents 3,4,5-trichlorothien-2-yl.

The present invention also provides a compound of formula 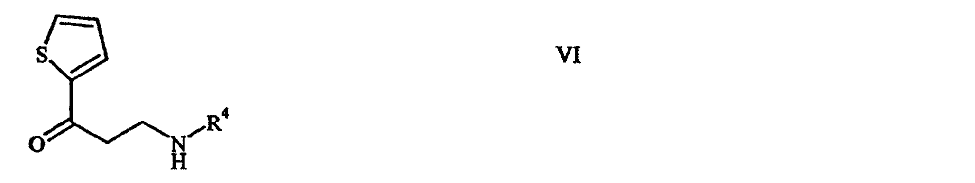 and its addition salts of proton acids, wherein R⁴ represents methyl, ethyl, isobutyl and *tert-*butyl.

The present invention also provides a compound of formula 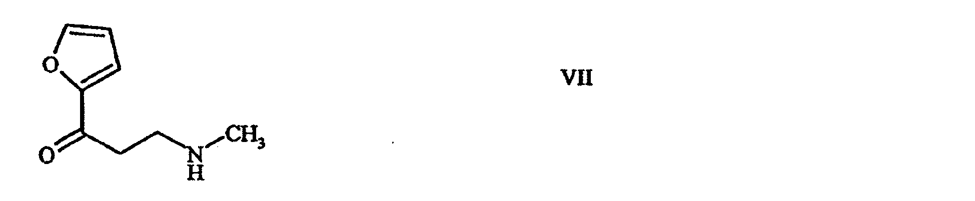 and its addition salts of proton acids.

The present invention also provides a compound of formula 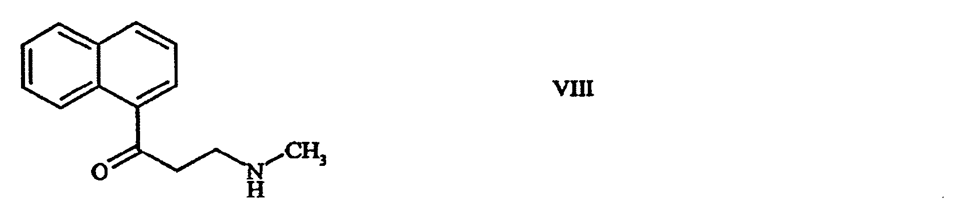 and its addition salts of proton acids.

The present invention is illustrated by the following non-limiting examples.

### General Procedure for Examples 1 to 8

A mixture of methyl ketone (1 equivalent (eq)), primary alkyl amine and/or an addition salt thereof (1.1 to 1.5 eq), formaldehyde (1.4 to 1.5 eq), a solvent, optionally in the presence of a proton acid, is heated in an autoclave at a total pressure above 1.5 bar for 5 to 24 hours. Afterwards, the reaction solution is cooled to 20 °C. Optionally the reaction solvent can than be removed partly or in whole and a solvent like ethyl acetate or isopropyl alcohol can be added under vigorous stirring, if necessary to facilitate precipitation of the product. The suspension is cooled (0 to 20 °C) and filtered after precipitation (0.5 to 10 hours), optionally washed and dried to afford a slightly yellow to white powder in a yield between 50 and 75 %. The product can be recrystallized from isopropyl alcohol and/or ethyl acetate if necessary. If the stability of the free base is sufficient at ambient conditions, extracting with an organic solvent and an aqueous base affords the free base.

### General Procedure for Comparative Examples 1 to 6

A mixture of methyl ketone (1 eq), primary alkyl amine and/or an addition salt thereof (1 to 1.5 eq), formaldehyde (1.0 to 1.5 eq), optionally in the presence of a proton acid, is heated in refluxing solvent for 5 to 24 hours. Afterwards, the mixture is cooled to 20 °C. Optionally the reaction solvent can than be removed partly or in whole and a solvent like ethyl acetate or isopropyl alcohol can be added under vigorous stirring, if necessary to facilitate precipitation of the product. The suspension is cooled (0 to 20 °C) and filtered after precipitation (0.5 to 10 hours), optionally washed and dried to afford a slightly yellow to white powder in a yield between 30 and 45 %. The product can be recrystallized from isopropyl alcohol and/or ethyl acetate if necessary.

### Example 1: 3-(Methylamino)-1-(thiophen-2-yl)propan-1-one hydrochloride (II, R¹ = thiophen-2-yl, R² = methyl)

2-Acetylthiophene (25.5 g, 200 mmol); methylamine hydrochloride (14.9 g, 220 mmol, 1.1 eq); paraformaldehyde (8.2 g, 280 mmol, 1.4 eq); HCl conc. (1.0 g); ethanol (100 mL); 110 °C for 9 hours; ca. 2 to 2.5 bar; removing of ethanol (50 mL) *in vacuo*; addition of ethyl acetate (200 mL); ca. 71 % yield.
¹H-NMR δ (DMSO-d₆, 400 MHz): 9.16 (2 H, s, br), 8.07 (1 H, dd, *J* = 5.0, 1.0), 8.01 (1 H, dd, *J* = 3.8, 1.0), 7.29 (1 H, dd, *J* = 5.0, 3.8), 3.49 (2 H, t), 3.20 (2 H, t), 2.56 (3 H, s). ¹³C-NMR δ (DMSO-d₆, 100 MHz): 189.9, 142.7, 135.4, 133.8, 128.8, 43.1, 34.6, 32.4.

### Example 2: 3-(Methylamino)-1-(thiophen-2-yl)propan-1-one hydrochloride (II, R¹ = thiophen-2-yl, R² = methyl)

2-Acetylthiophene (24.9 g, 197 mmol); methylamine hydrochloride (14.8 g, 219 mmol, 1.1 eq); paraformaldehyde (8.3 g, 276 mmol, 1.4 eq); HCl conc. (1.1 g); isopropyl alcohol (100 mL); 110 °C for 8 hours; ca. 2 to 2.5 bar; addition of isopropyl alcohol (50 mL); ca. 65 % yield.

### Comparative Example 1: 3-(Methylamino)-1-(thiophen-2-yl)propan-1-one hydrochloride (II, R¹ = thiophen-2-yl, R² = methyl)

2-Acetylthiophene (7.9 g, 300 mmol); methylamine hydrochloride (30.4 g, 450 mmol, 1.5 eq); paraformaldehyde (12.6 g, 420 mmol, 1.4 eq); HCl conc. (1.5 g); isopropyl alcohol (200 mL); heating under reflux (82 °C) for 8 hours; addition of ethyl acetate (200 mL); ca. 43 % yield.

### Example 3: 3-(Ethylamino)-1-(thiophen-2-yl)propan-1-one hydrochloride (II, R¹ = thiophen-2-yl, R² = ethyl)

2-Acetylthiophene (6.3 g, 50 mmol); ethylamine hydrochloride (6.1 g, 75 mmol, 1.5 eq); paraformaldehyde (2.1 g, 75 mmol, 1.5 eq); HCl conc. (0.3 g); ethanol (35 mL); 110 °C for 9 hours; ca. 2 to 2.5 bar; removing of ethanol (25 mL) *in vacuo*; addition of ethyl acetate (50 mL); ca. 73 % yield.
¹H-NMR δ (DMSO-d₆, 400 MHz): 9.3 (2 H, s, br), 8.08 (1 H, dd), 8.00 (1 H, dd), 7.28 (1 H, dd), 3.51 (2 H, t), 3.20 (2 H, t), 2.96 (2 H, q), 1.23 (3 H, t).

### Comparative Example 2: 3-(Ethylamino)-1-(thiophen-2-yl)propan-1-one hydrochloride (II, R¹ = thiophen-2-yl, R² = ethyl)

2-Acetylthiophene (12.6 g, 100 mmol); ethylamine hydrochloride (12.2 g, 150 mmol, 1.5 eq); paraformaldehyde (4.1 g, 140 mmol, 1.4 eq); HCl conc. (0.5 g); ethanol (70 mL); heating under reflux (78 °C) for 6 hours; removing of ethanol (25 mL) *in vacuo*; addition of ethyl acetate (70 mL); ca. 31 % yield.

### Example 4: 3-(Isobutylamino)-1-(thiophen-2-yl)propan-1-one hydrochloride (II, R¹ = thiophen-2-yl, R² = isobutyl)

2-Acetylthiophene (6.3 g, 50 mmol); isobutylamine hydrochloride (8.3 g, 75 mmol, 1.5 eq); paraformaldehyde (2.1 g, 75 mmol, 1.5 eq); HCl conc. (0.3 g); ethanol (35 mL); 110 °C for 9 hours; ca. 2 to 2.5 bar; removing of ethanol (35 mL) *in vacuo*; addition of ethyl acetate (50 mL); ca. 56 % yield.
¹H-NMR δ (DMSO-d₆, 400 MHz): 9.0 (2 H, s, br), 8.08 (1 H, dd), 7.99 (1 H, dd), 7.29 (1 H, dd), 3.55 (2 H, t), 3.22 (2 H, t), 2.78 (2 H, d), 2.03 (1 H, m), 0.96 (6 H, d).

### Comparative Example 3: 3-(Isobutylamino)-1-(thiophen-2-yl)propan-1-one hydrochloride (II, R¹ = thiophen-2-yl, R² = isobutyl)

2-Acetylthiophene (12.6 g, 100 mmol); isobutylamine hydrochloride (16.5 g, 150 mmol, 1.5 eq); paraformaldehyde (4.1 g, 140 mmol, 1.4 eq); HCl conc. (0.5 g); butanol (70 mL); heating under reflux (108 °C) for 7 hours; addition of ethyl acetate (100 mL); ca. 40 % yield.

### Example 5: 3-(tert-Butylamino)-1-(thiophen-2-yl)propan-1-one hydrochloride (II, R¹ = thiophen-2-yl, R² = tert-butyl)

2-Acetylthiophene (6.3 g, 50 mmol); *tert*-butylamine hydrochloride (8.3 g, 75 mmol, 1.5 eq); paraformaldehyde (2.1 g, 75 mmol, 1.5 eq); HCl conc. (0.3 g); butanol (35 mL); 117 °C for 9 hours; ca. 2 to 2.5 bar; addition of ethyl acetate (50 mL); ca. 52 % yield.
¹H-NMR δ (DMSO-d₆, 400 MHz): 9.2 (2 H, s, br), 8.08 (1 H, dd), 7.98 (1 H, dd), 7.30 (1 H, dd), 3.54 (2 H, t), 3.19 (2 H, t), 1.34 (9 H, s).

### Comparative Example 4: 3-(tert-Butylamino)-1-(thiophen-2-yl)propan-1-one hydrochloride (II, R¹ = thiophen-2-yl, R² = tert-butyl)

2-Acetylthiophene (12.6 g, 100 mmol); *tert*-butylamine hydrochloride (16.5 g, 150 mmol, 1.5 eq); paraformaldehyde (4.1 g, 140 mmol, 1.4 eq); HCl conc. (0.5 g); butanol (70 mL); heating under reflux (108 °C) for 18 hours; addition of ethyl acetate (100 mL); ca. 37 % yield.

### Example 6: 3-(Methylamino)-1-(furan-2-yl)propan-1-one hydrochloride (II, R¹ = furan-2-yl, R² = methyl)

2-Acetylfuran (7.5 g, 68 mmol); methylamine hydrochloride (6.9 g, 102 mmol, 1.5 eq); paraformaldehyde (3.1 g, 102 mmol, 1.5 eq); HCl conc. (1.15 g); ethanol (35 mL); 110 °C for 8 hours; ca. 2 to 2.5 bar; removing of ethanol (30 mL) *in vacuo*; addition of ethyl acetate (50 mL); ca. 64 % yield.
¹H-NMR δ (DMSO-d₆, 400 MHz): 9.0 (2 H, s, br), 8.05 (1 H, m), 7.53 (1 H, m), 6.77 (1 H, m), 3.34 (2 H, t), 3.2 (2 H, m), 2.57 (3 H, s, br).

### Comparative Example 5: 3-(Methylamino)-1-(furan-2-yl)propan-1-one hydrochloride (II, R¹ = furan-2-yl, R² = methyl)

2-Acetylfuran (11.0 g, 100 mmol); methylamine hydrochloride (10.1 g, 150 mmol, 1.5 eq); paraformaldehyde (4.1 g, 140 mmol, 1.4 eq); HCl conc. (0.5 g); butanol (70 mL); heating under reflux (108 °C) for 7 hours; addition of ethyl acetate (100 mL); ca. 44 % yield.

### Example 7: 3-(Methylamino)-1-phenylpropan-1-one hydrochloride (II, R¹ = phenyl, R² = methyl)

2-Acetophenone (21.0 g, 175 mmol); methylamine hydrochloride (17.5 g, 263 mmol, 1.5 eq); paraformaldehyde (7.9 g, 263 mmol, 1.5 eq); HCl conc. (1.1 g); ethanol (130 mL); 115 °C for 24 hours; ca. 2 to 2.5 bar; addition of ethyl acetate (170 mL); ca. 52 % yield.
¹H-NMR δ (DMSO-d₆, 400 MHz): 9.2 (2 H, s, br), 8.0 (2 H, m), 7.7 (1 H, m), 7.6 (2 H, m), 3.55 (2 H, t), 3.21 (2 H, t), 2.59 (3 H, s).

### Example 8: 3-(Methylamino)-1-(2-naphthyl)propan-1-one hydrochloride (II, R¹ = 2-naphthyl, R² = methyl)

2-Acetonaphtone (8.5 g, 50 mmol); methylamine hydrochloride (5.1 g, 75 mmol, 1.5 eq); paraformaldehyde (2.1 g, 75 mmol, 1.5 eq); HCl conc. (0.3 g); ethanol (35 mL); 117 °C for 14 hours; ca. 2 to 2.5 bar; removing of ethanol (35 mL) *in vacuo*; addition of ethyl acetate (50 mL); ca. 60 % yield.
¹H-NMR δ (DMSO-d₆, 400 MHz): 9.3 (2 H, s, br), 8.74 (1 H, s), 8.17 (1 H, d), 8.0 (3 H, m), 7.7 (2 H, m), 3.70 (2 H, t), 3.28 (2 H, m), 2.60 (3 H, s).

### Comparative Example 6: 3-(Methylamino)-1-(2-naphthyl)propan-1-one hydrochloride (II, R¹ = 2-naphthyl, R² = methyl)

2-Acetonaphtone (17.0 g, 100 mmol); methylamine hydrochloride (10.1 g, 150 mmol, 1.5 eq); paraformaldehyde (4.1 g, 140 mmol, 1.4 eq); HCl conc. (0.5 g); ethanol (70 mL); heating under reflux (78 °C) for 5 hours; removing of ethanol (30 mL) *in vacuo*; addition of ethyl acetate (100 mL); ca. 42 % yield.

### Example 9: 3-(Methylamino)-1-(thiophen-2-yl)propan-1-ol (I, R¹ = thiophen-2-yl, R² = methyl)

To a mixture of 3-(methylamino)-1-(thiophen-2-yl)propan-1-one hydrochloride (10.3 g, 50 mmol) and ethanol (35 mL) at 4 °C sodium hydroxide (4.0 g of a 50 % aqueous solution) was added in about 5 minutes. Afterwards, neat sodium borhydride (0.95 g, 25 mmol, 1.0 eq) was added in several portions in about 30 minutes. At the end of the addition, the suspension was stirred for 4 h at the same temperature, then acetone (10.0 mL) was added dropwise in 5 minutes and the mixture was stirred for 10 additional minutes. Water (20 mL) was then added. Afterwards, the mixture was concentrated about 5 times under vacuum and the residue was extracted with tert-butyl methyl ether (2 x 20 mL). The collected organic phases were finally concentrated under vacuum affording an orange oil which crystallised spontaneously after a few hours. Finally, an orange solid was obtained (7.2 g, 84 % yield). This compound can then be used without further purification.
¹H-NMR δ (DMSO-d₆, 400 MHz): 7.35 (1 H, dd, J = 4.8, 1.0), 6.94 (1 H, dd, J = 4.8, 3.6), 6.90 (1 H, dd, J = 3.6, 1.0), 4.90 (1 H, t), 3.7 (2 H, m), 2.56 (2 H, m), 2.25 (3 H, s), 1.79 (2 H, q).
¹³C-NMR δ (DMSO-d₆, 100 MHz): 150.9, 126.3, 123.7, 122.3, 67.8, 48.5, 38.7, 36.0.

### Example 10: 3-(Isobutylamino)-1-(thiophen-2-yl)propan-1-ol (I, R¹ = thiophen-2-yl, R² = methyl)

To a mixture of 3-(isobutylamino)-1-(thiophen-2-yl)propan-1-one hydrochloride (4.2 g, 19.4 mmol) and ethanol (10 mL) at 4 °C sodium hydroxide (1.6 g of a 50 % aqueous solution) was added in about 20 minutes. Afterwards, neat sodium borhydride (0.37 g, 9.7 mmol, 1.0 eq) was added in several portions in about 30 minutes. At the end of the addition, the suspension was stirred for 4 h at the same temperature, then acetone (10.0 mL) was added dropwise in 20 minutes and the mixture was stirred for 10 additional minutes. Afterwards the precipitate was removed by filtration and the mixture was concentrated under vacuum affording an orange oil. The crude product was purified by column chromatography using a 40 : 10 : 1 (v : v : v) mixture of methylene chloride/methanol/ammonium hydroxide (25 % aqueous solution) affording 3.1 g (76 % yield) of product.
¹H-NMR δ (DMSO-d₆, 400 MHz): 7.20 (1 H, dd, J = 4.8, 1.0), 6.98 (1 H, dd), 6.94 (1 H, dd, J = 4.8, 3.6), 5.20 (1 H, dd), 4.98 (2 H, br), 3.02 (1 H, m), 2.93 (1 H, m), 2.43 (2H, symm. m), 2.03 (1 H, m), 1.97 (1 H, m), 1.80 (1 H, sept), 0.95 (6 H, d).
¹³C-NMR δ (DMSO-d₆, 100 MHz): 150.9, 126.3, 123.8, 122.5, 72.1, 57.8, 48.5, 37.4, 28.2, 20.8.

## Claims

1. A process for the preparation of a compound of formula 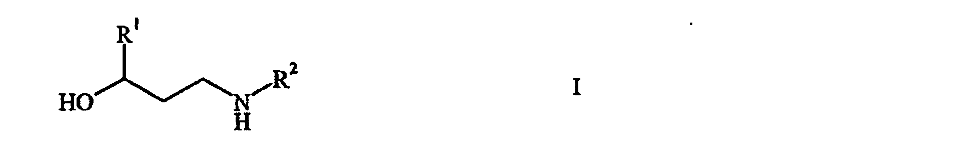 and/or an addition salt of a proton acid, wherein R¹ and R² independently represent alkyl, cycloalkyl, aryl or aralkyl, each aryl or aralkyl being optionally further substituted with alkyl, alkoxy and/or halogen, which process comprises the following steps
a) reacting a mixture comprising
(i) a methyl ketone of formula 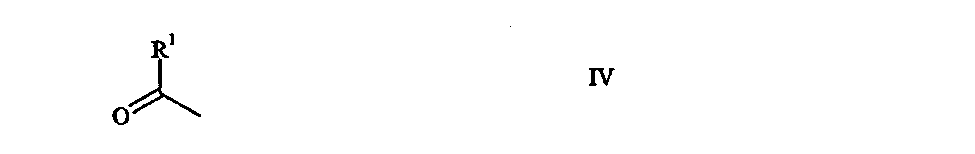 wherein R¹ is as defined above, and
(ii) a compound of formula
H₂N-R² V
and/or an addition salt of proton acid, wherein R² is as defined above, and
(iii) formaldehyde or a source of formaldehyde selected from the group consisting of formaldehyde in aqueous solution, 1,3,5-trioxane, paraformaldehyde and mixtures thereof, in the presence of
a solvent selected from the group consisting of water, aliphatic alcohols, cycloaliphatic alcohols and mixtures thereof, and
optionally a proton acid
to afford a β-keto amine of formula 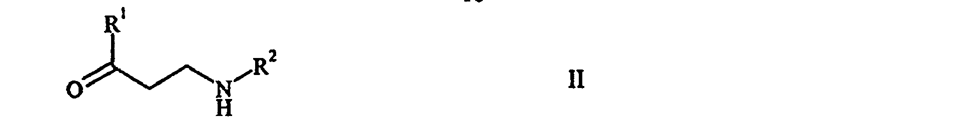 and/or an addition salt of a proton acid, and
b) reducing the carbonyl group of said β-keto amine to afford a compound of formula I, and/or an addition salt of a proton acid
wherein the first step is carried out at a pressure above 1.5 bar.

2. The process of claim 1 wherein R¹ is selected from the group consisting of linear or branched C₁₋₈ alkyl, C₃₋₈ cycloalkyl, phenyl, naphthyl, furanyl, benzofuranyl, thienyl, benzo[b]thienyl and aralkyl, wherein the alkyl moiety of the aralkyl residue is linear C₁₋₄ alkyl, and the aryl moiety is selected from the group consisting of phenyl, naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅.

3. The process of claim 1 or 2 wherein R² is selected from the group consisting of linear or branched C₁₋₈ alkyl, C₃₋₈ cycloalkyl, phenyl, naphthyl, furanyl, benzofuranyl, thienyl, benzo[b]thienyl and aralkyl, wherein the alkyl moiety of the aralkyl residue is linear C₁₋₄ alkyl, and the aryl moiety is selected from the group consisting of phenyl, naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅.

4. The process of any of claims 1 to 3, wherein the compound of formula V is present in an amount at least equimolar to that of the compound of formula IV.

5. The process of any of claims 1 to 4, wherein the proton acid is a carboxylic or an inorganic acid, the acid being preferably selected from the group consisting of formic acid, acetic acid, propionic acid, oxalic acid, malonic acid, benzoic acid, HF, HCl, HBr, HI, H₂SO₄, H₃PO₄, mono alkali malonate, alkali hydrogensulfates, alkali hydrogenphosphates and alkali hydrogencarbonates.

6. The process of any of claims 1 to 5, wherein aliphatic and cycloaliphatic alcohols are selected from the group selected of linear or branched aliphatic C₁₋₁₂ alcohols, cycloaliphatic C₅₋₈ alcohols, di- and/or triethylene glycols and mono C₁₋₄ alkyl or acetyl derivatives thereof, each of said alcohols containing 1 to 3 hydroxy groups.

7. The process of claim 6, wherein the alcohol is selected from the group consisting of methanol, ethanol, propanol, isopropyl alcohol, butanol, isobutanol, *tert*-butanol, 1-pentanol, 2-pentanol, 3-pentanol, 1-hexaaol, 2-hexanol, cyclopentanol, cyclohexanol, 1,2-ethanediol, 1,2-propanediol, 1,2-butanediol, 2,3-butanediol, 1,4-butanediol, 1,2,3-propanetriol, 1,2,6-hexanetriol, diethylene glycol, diethylene glycol monomethyl ether, diethylene glycol monoethyl ether, diethylene glycol monobutyl ether, diethylene glycol monoacetate, triethylene glycol, triethylene glycol monomethyl ether, triethylene glycol monoethyl ether, triethylene glycol monobutyl ether and triethylene glycol monoacetate.

8. The process of any of claims 1 to 7, wherein the pressure during reaction step a) is above 1.5 bar.

9. A compound of formula 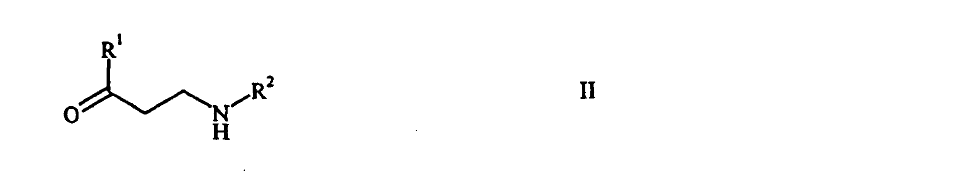 and its addition salts of proton acids, wherein R¹ represents furanyl, benzofuranyl, isobenzofuranyl, thienyl or benzo[b]thienyl,
each being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅; and
wherein R² is selected from the group consisting of linear or branched C₁₋₈ alkyl, C₃₋₈ cycloalkyl, furanyl, benzofuranyl, thienyl, benzo[b]thienyl and aralkyl, wherein the alkyl moiety of the aralkyl residue is linear C₁₋₄ alkyl, and the aryl moiety is selected from the group consisting of naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅ with the exception of compounds wherein R¹ represents 3,4,5-trichlorothien-2-yl.

10. A compound of formula 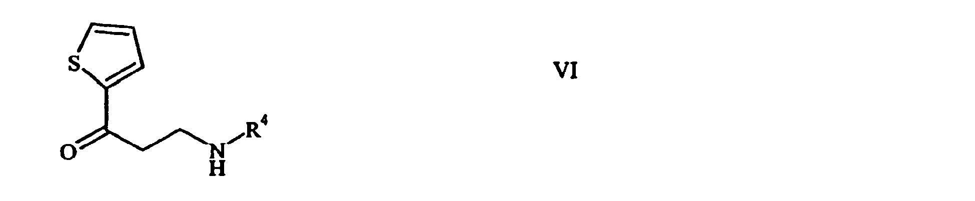 and its addition salts of proton acids, wherein R⁴ represents methyl, ethyl, isobutyl or *tert-*butyl.

11. A compound of formula 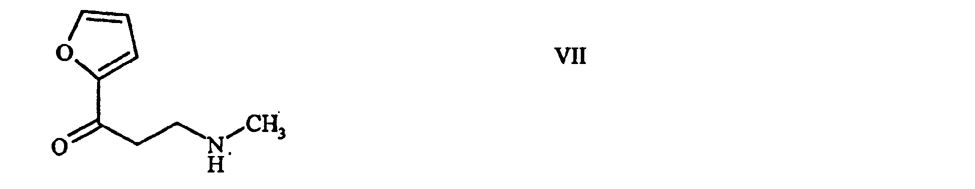 and its addition salts of proton acids.

12. A compound of formula 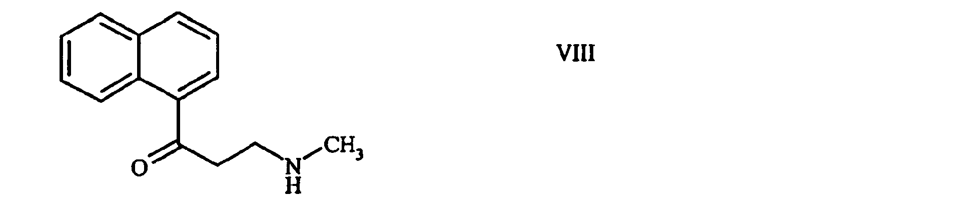 and its addition salts of proton acids.

## Patentansprüche

1. Verfahren zur Herstellung einer Verbindung der Formel 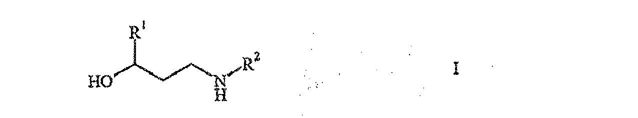 und/oder eines Protonensäureadditionssalzes, worin R¹ und R² unabhängig voneinander für Alkyl, Cycloalkyl, Aryl oder Arylakyl stehen, wobei jedes Aryl oder Aralkyl gegebenenfalls ferner durch Alkyl, Alkoxy und/oder Halogen substituiert ist, bei dem man in den Schritten
a) eine Mischung, enthaltend
(i) ein Methylketon der Formel 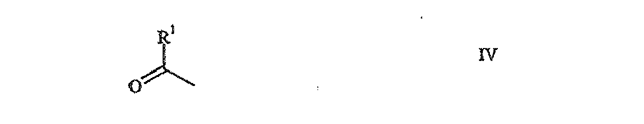 worin R¹ die oben angegebene Bedeutung besitzt, und
(ii) eine Verbindung der Formel
H₂N-R² V
und/oder ein Protonensäureadditionssalz, worin R² die oben angegebene Bedeutung besitzt, und
(iii) Formaldehyd oder eine aus der Gruppe bestehend aus Formaldehyd in wäßriger Lösung, 1,3,5-Trioxan, Paraformaldehyd und Mischungen davon ausgewählte Formaldehyd-Quelle, in Gegenwart
eines Lösungsmittels aus der Gruppe bestehend aus Wasser, aliphatischen Alkoholen, cycloaliphatischen Alkoholen und Mischungen davon und
gegebenenfalls einer Protonensäure
zu einem β-Ketoamin der Formel 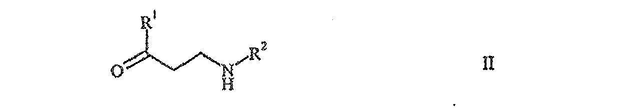 und/oder einem Protonensäureadditionssalz umsetzt und
b) die Carbonylgruppe des β-Ketoamins reduziert, wobei man eine Verbindung der Formel I und/oder ein Protonensäureadditionssalz erhält,
wobei man den ersten Schritt bei einem Druck über 1,5 bar durchführt.

2. Verfahren nach Anspruch 1, bei dem R¹ aus der Gruppe bestehend aus linearem oder verzweigtem C₁₋₈-Alkyl, C₃₋₈-Cycloalkyl, Phenyl, Naphthyl, Furanyl, Benzofuranyl, Thienyl, Benzo[b]thienyl und Aralkyl stammt, wobei der Alkylteil des Aralkylrests lineares C₁₋₄Alkyl ist und der Arylteil aus der Gruppe bestehend aus Phenyl, Naphthyl, Furanyl, Benzofuranyl, Thienyl und Benzo[b]thienyl stammt,
wobei jedes Aryl oder Aralkyl gegebenenfalls durch Halogen, lineares oder verzweigtes C₁₋₄-Alkyl, lineares oder verzweigtes C₁₋₄-Alkoxy, C₃₋₆-Cycloalkyl, CF₃, C₂F₅, OCF₃ oder OC₂F₅ substituiert ist.

3. Verfahren nach Anspruch 1 oder 2, bei dem R² aus der Gruppe bestehend aus linearem oder verzweigtem C₁₋₈-Alkyl, C₃₋₈-Cycloalkyl, Phenyl, Naphthyl, Furanyl, Benzofuranyl, Thienyl, Benzo[b]thienyl und Aralkyl stammt, wobei der Alkylteil des Aralkylrests lineares C₁₋₄-Alkyl ist und der Arylteil aus der Gruppe bestehend aus Phenyl, Naphthyl, Furanyl, Benzofuranyl, Thienyl und Benzo[b]thienyl stammt,
wobei jedes Aryl oder Aralkyl gegebenenfalls durch Halogen, lineares oder verzweigtes C₁₋₄-Alkyl, lineares oder verzweigtes C₁₋₄-Alkoxy, C₃₋₆-Cycloalkyl, CF₃, C₂F₅, OCF₃ oder OC₂F₅ substituiert ist.

4. Verfahren nach einem der Ansprüche 1 bis 3, bei dem die Verbindung der Formel V in einer zur Menge der Verbindung der Formel IV zumindest äquimolaren Menge vorliegt.

5. Verfahren nach einem der Ansprüche 1 bis 4, bei dem es sich bei der Protonensäure um eine Carbonsäure oder eine anorganische Säure handelt, die vorzugsweise aus der Gruppe bestehend aus Ameisensäure, Essigsäure, Propionsäure, Oxalsäure, Malonsäure, Benzoesäure, HF, HCl, HBr, HI, H₂SO₄, H₃PO₄, Monoalkalimalonat, Alkalihydrogensulfaten, Alkalihydrogenphosphaten und Alkalihydrogencarbonaten stammt.

6. Verfahren nach einem der Ansprüche 1 bis 5, bei dem aliphatische und cycloaliphatische Alkohole aus der Gruppe bestehend aus linearen oder verzweigten C₁₋₁₂-Alkoholen, cycloaliphatischen C₅₋₈-Alkoholen, Di- und/oder Triethylenglykolen und Mono-C₁₋₄- oder Acetylderivaten davon stammen, wobei jeder der Alkohole 1 bis 3 Hydroxygruppen enthält.

7. Verfahren nach Anspruch 6, bei dem der Alkohol aus der Gruppe bestehend aus Methanol, Ethanol, Propanol, Isopropylalkohol, Butanol, Isobutanol, *tert*-Butanol, 1-Pentanol, 2-Pentanol, 3-Pentanol, 1-Hexanol, 2-Hexanol, Cyclopentanol, Cyclohexanol, 1,2-Ethandiol, 1,2-Propandiol, 1,2-Butandiol, 2,3-Butandiol, 1,4-Butandiol, 1,2,3-Propantriol, 1,2,6-Hexantriol, Diethylenglykol, Diethylenglykolmonomethylether, Diethylenglykolmonoethylether, Diethylenglykolmonobutylether, Diethylenglykolmonoacetat, Triethylenglykol, Triethylenglykolmonomethylether, Triethylenglykolmonoethylether, Triethylenglykolmonobutylether und Triethylenglykolmonoacetat stammt.

8. Verfahren nach einem der Ansprüche 1 bis 7, bei dem der Druck während Reaktionsschritt a) über 1,5 bar beträgt.

9. Verbindung der Formel 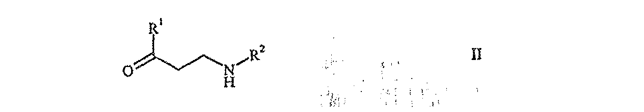 und ihre Protonensäureadditionssalze, worin R¹ für Furanyl, Benzofuranyl, Isobenzofuranyl, Thienyl oder Benzo[b]thienyl steht, wobei jede dieser Gruppen gegebenenfalls durch Halogen, lineares oder verzweigtes C₁₋₄-Alkyl, lineares oder verzweigtes C₁₋₄-Alkoxy, C₃₋₆-Cycloalkyl, CF₃, C₂F₅, OCF₃ oder OC₂F₅ substituiert ist und R² aus der Gruppe bestehend aus linearem oder verzweigtem C₁₋₈-Alkyl, C₃₋₈-Cycloalkyl, Furanyl, Benzofuranyl, Thienyl, Benzo[b]thienyl und Aralkyl stammt, wobei der Alkylteil des Aralkylrests lineares C₁₋₄-Alkyl ist und der Arylteil aus der Gruppe bestehend aus Naphthyl, Furanyl, Benzofuranyl, Thienyl und Benzo[b]thienyl stammt,
wobei jedes Aryl oder Aralkyl gegebenenfalls durch Halogen, lineares oder verzweigtes C₁₋₄-Alkyl, lineares oder verzweigtes C₁₋₄-Alkoxy, C₃₋₆-Cycloalkyl, CF₃, C₂F₅, OCF₃ oder OC₂F₅ substituiert ist,
mit Ausnahme der Verbindungen, in denen R¹ für 3,4,5-Trichlorthien-2-yl steht.

10. Verbindung der Formel 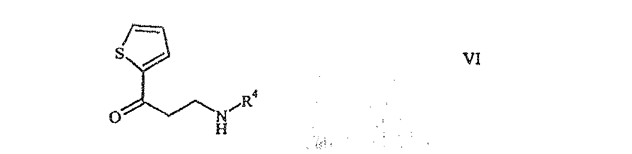 und ihre Protonensäureadditionssalze, worin R⁴ für Methyl, Ethyl, Isobutyl oder *tert*-Butyl steht.

11. Verbindung der Formel 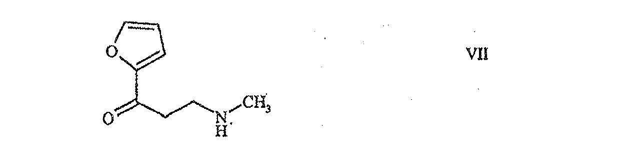 und ihre Protonensäureadditionssalze.

12. Verbindung der Formel 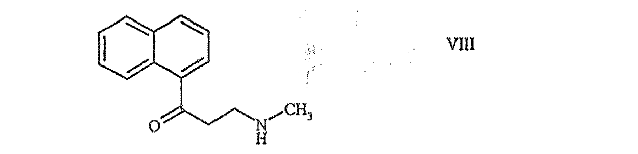 und ihre Protonensäureadditionssalze.

## Revendications

1. Procédé pour la préparation d'un composé de formule 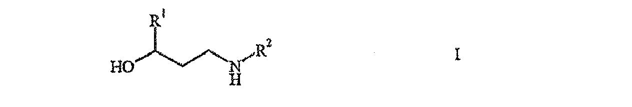 et/ou d'un sel d'addition d'un acide protonique, dans laquelle R¹ et R² représentent indépendamment un groupe alkyle, un groupe cycloalkyle, un groupe aryle ou un groupe aralkyle, chaque groupe aryle ou aralkyle étant en outre éventuellement substitué par un groupe alkyle, un groupe alcoxy et/ou un atome d'halogène, lequel procédé comprend les étapes suivantes :
a) la réaction d'un mélange comprenant :
(i) une méthylcétone de formule 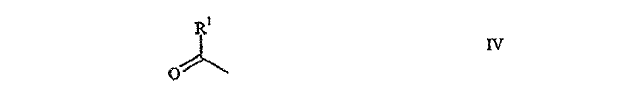 dans laquelle R¹ est tel que défini ci-dessus, et
(ii) un composé de formule
**H₂N-R²** **V**
et/ou un sel d'addition d'un acide protonique, dans laquelle R² est tel que défini ci-dessus, et
(iii) du formaldéhyde ou une source de formaldéhyde choisie parmi le groupe constitué du formaldéhyde en solution aqueuse, du 1,3,5-trioxane, du para-formaldéhyde et de leurs mélanges, en présence
d'un solvant choisi parmi le groupe constitué de l'eau, d'alcools aliphatiques, d'alcools cycloaliphatiques et de leurs mélanges, et
éventuellement, d'un acide protonique,
pour donner lieu à une β-cétoamine de formule 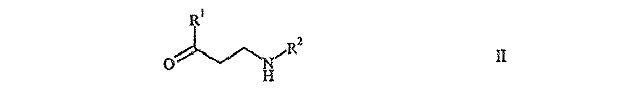 et/ou un sel d'addition d'un acide protonique, et
b) la réduction du groupe carbonyle de ladite β-cétoamine pour donner lieu à un composé de formule I, et/ou un sel d'addition d'un acide protonique,
où la première étape est réalisée à une pression supérieure à 1,5 bar.

2. Procédé selon la revendication 1, dans lequel R¹ est choisi parmi le groupe constitué d'un groupe alkyle en C₁₋₈ linéaire ou ramifié, d'un groupe cycloalkyle en C₃₋₈, d'un groupe phényle, d'un groupe naphtyle, d'un groupe furanyle, d'un groupe benzofuranyle, d'un groupe thiényle, d'un groupe benzo[b]thiényle et d'un groupe aralkyle, où le fragment alkyle du résidu aralkyle est un groupe alkyle en C₁₋₄ linéaire, et le fragment aryle est choisi parmi le groupe constitué d'un groupe phényle, d'un groupe naphtyle, d'un groupe furanyle, d'un groupe benzofuranyle, d'un groupe thiényle et d'un groupe benzo[b]thiényle,
chaque groupe aryle ou aralkyle étant éventuellement substitué par un atome d'halogène, un groupe alkyle en C₁₋₄ linéaire ou ramifié, un groupe alcoxy en C₁₋₄ linéaire ou ramifié, un groupe cycloalkyle en C₃₋₆, un groupe CF₃, un groupe C₂F₅, un groupe OCF₃ ou un groupe OC₂F₅.

3. Procédé selon la revendication 1 ou 2, dans lequel R² est choisi parmi le groupe constitué d'un groupe alkyle en C₁₋₈ linéaire ou ramifié, d'un groupe cycloalkyle en C₃₋₈, d'un groupe phényle, d'un groupe naphtyle, d'un groupe furanyle, d'un groupe benzofuranyle, d'un groupe thiényle, d'un groupe benzo[b]thiényle et d'un groupe aralkyle, où le fragment alkyle du résidu aralkyle est un groupe alkyle en C₁₋₄ linéaire, et le fragment aryle est choisi parmi le groupe constitué d'un groupe phényle, d'un groupe naphtyle, d'un groupe furanyle, d'un groupe benzofuranyle, d'un groupe thiényle et d'un groupe benzo[b]thiényle,
chaque groupe aryle ou aralkyle étant éventuellement substitué par un atome d'halogène, un groupe alkyle en C₁₋₄ linéaire ou ramifié, un groupe alcoxy en C₁₋₄ linéaire ou ramifié, un groupe cycloalkyle en C₃₋₆, un groupe CF₃, un groupe C₂F₅, un groupe OCF₃ ou un groupe OC₂F₅.

4. Procédé selon l'une quelconque des revendications 1 à 3, dans lequel le composé de formule V est présent en une quantité au moins équimolaire par rapport du celle du composé de formule IV.

5. Procédé selon l'une quelconque des revendications 1 à 4, dans lequel l'acide protonique est un acide carboxylique ou un acide inorganique, l'acide étant de préférence choisi parmi le groupe constitué de l'acide formique, de l'acide acétique, de l'acide propionique, de l'acide oxalique, de l'acide malonique, de l'acide benzoïque, du HF, du HCl, du HBr, du HI, du H₂SO₄, du H₃PO₄, d'un malonate mono-alcalin, d'hydrogéno-sulfates alcalins, d'hydrogénophosphates alcalins et d'hydrogénocarbonates alcalins.

6. Procédé selon l'une quelconque des revendications 1 à 5, dans lequel les alcools aliphatiques et cycloaliphatiques sont choisis parmi le groupe constitué d'alcools en C₁₋₁₂ aliphatiques linéaires ou ramifiés, d'alcools en C₅₋₈ cycloaliphatiques, de di- et/ou de tri-éthylèneglycols et de leurs dérivés mono-C₁₋₄-alkyliques ou acétyliques, chacun desdits alcools renfermant de 1 à 3 groupes hydroxy.

7. Procédé selon la revendication 6, dans lequel l'alcool est choisi parmi le groupe constitué du méthanol, de l'éthanol, du propanol, de l'alcool isopropylique, du butanol, de l'isobutanol, du tert-butanol, du 1-pentanol, du 2-pentanol, du 3-pentanol, du 1-hexanol, du 2-hexanol, du cyclopentanol, du cyclohexanol, du 1,2-éthanediol, du 1,2-propanediol, du 1,2-butanediol, du 2,3-butanediol, du 1,4-butanediol, du 1,2,3-propanetriol, du 1,2,6-hexanetriol, du diéthylèneglycol, de l'éther monométhylique de diéthylèneglycol, de l'éther monoéthylique de diéthylèneglycol, de l'éther monobutylique de diéthylèneglycol, du monoacétate de diéthylèneglycol, du triéthylèneglycol, de l'éther monométhylique de triéthylèneglycol, de l'éther monoéthylique de triéthylèneglycol, de l'éther monobutylique de triéthylèneglycol et du monoacétate de triéthylèneglycol.

8. Procédé selon l'une quelconque des revendications 1 à 7, dans lequel la pression durant l'étape de réaction a) est supérieure à 1,5 bar.

9. Composé de formule 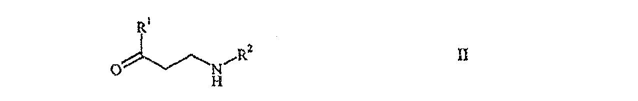 et ses sels d'addition d'acides protoniques,
dans laquelle R¹ représente un groupe furanyle, un groupe benzofuranyle, un groupe isobenzofuranyle, un groupe thiényle ou un groupe benzo[b]thiényle, chacun étant éventuellement substitué par un atome d'halogène, un groupe alkyle en C₁₋₄ linéaire ou ramifié, un groupe alcoxy en C₁₋₄ linéaire ou ramifié, un groupe cycloalkyle en C₃₋₆, un groupe CF₃, un groupe C₂F₅, un groupe OCF₃ ou un groupe OC₂F₅ ; et
dans laquelle R² est choisi parmi le groupe constitué d'un groupe alkyle en C₁₋₈ linéaire ou ramifié, d'un groupe cycloalkyle en C₃₋₈, d'un groupe furanyle, d'un groupe benzofuranyle, d'un groupe thiényle, d'un groupe benzo[b]thiényle et d'un groupe aralkyle, où le fragment alkyle du résidu aralkyle est un groupe alkyle en C₁₋₄ linéaire, et le fragment aryle est choisi parmi le groupe constitué d'un groupe naphtyle, d'un groupe furanyle, d'un groupe benzofuranyle, d'un groupe thiényle et d'un groupe benzo[b]thiényle,
chaque groupe aryle ou aralkyle étant éventuellement substitué par un atome d'halogène, un groupe alkyle en C₁₋₄ linéaire ou ramifié, un groupe alcoxy en C₁₋₄ linéaire ou ramifié, un groupe cycloalkyle en C₃₋₆, un groupe CF₃, un groupe C₂F₅, un groupe OCF₃ ou un groupe OC₂F₅, excepté les composés dans lesquels R¹ représente un groupe 3,4,5-trichlorothién-2-yle.

10. Composé de formule 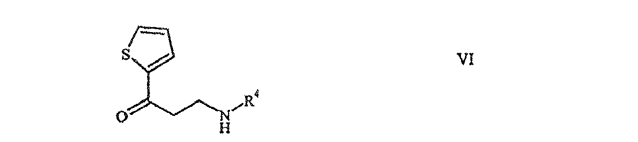 et ses sels d'addition d'acides protoniques, dans laquelle R⁴ représente un groupe méthyle, un groupe éthyle, un groupe isobutyle ou un groupe tert-butyle.

11. Composé de formule 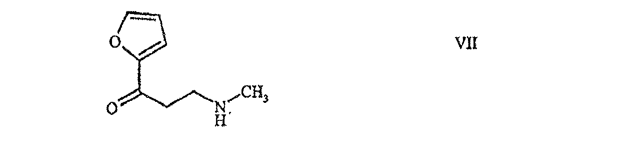 et ses sels d'addition d'acides protoniques.

12. Composé de formule 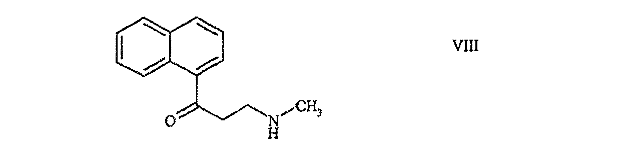 et ses sels d'addition d'acides protoniques.
